(19) Europäisches Patentamt
European Patent Office
Office européen des brevets

(11) **EP 4 223 866 A1**

(12) **EUROPEAN PATENT APPLICATION**

(43) Date of publication:
**09.08.2023 Bulletin 2023/32**

(21) Application number: **22305119.4**

(22) Date of filing: **03.02.2022**

(51) International Patent Classification (IPC):
*C12M 1/34* (2006.01)    *C12M 3/06* (2006.01)
*C12N 1/20* (2006.01)    *C12Q 1/04* (2006.01)
*C12Q 1/6888* (2018.01)    *G01N 33/569* (2006.01)

(52) Cooperative Patent Classification (CPC):
**C12Q 1/04; C12M 1/3476; C12M 23/16; C12N 1/20;
C12Q 1/6888; G01N 33/569**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Centre national de la recherche scientifique
75016 Paris (FR)**

• **Université de Strasbourg
67000 Strasbourg (FR)**

(72) Inventors:
• **RYCKELYNCK, Michaël
67000 STRASBOURG (FR)**
• **GEERSENS, Emilie
67000 STRASBOURG (FR)**

(74) Representative: **Gevers & Orès
Immeuble le Palatin 2
3 Cours du Triangle
CS 80165
92939 Paris La Défense Cedex (FR)**

(54) **METHOD FOR MICROFLUIDIC QUANTIFICATION, GROWTH MONITORING AND SEPARATION OF MICROORGANISMS IN DROPLETS**

(57)    The present invention relates to a method for detecting and/or quantifying at least one microorganism in a sample by encapsulating the sample with a fluorescent dye into water-in-oil emulsion droplets using a microfluidic device, incubating the droplets to allow at least one microorganism to grow, measuring the fluorescence in each droplet, and detecting and/or quantifying at least one population of droplets comprising a microorganism. The method of the invention further allows sorting the droplets depending on their fluorescence intensity and thus separating each microorganism.

EP 4 223 866 A1

**Description**

The present invention relates to a method for detecting and/or quantifying at least one microorganism in a sample by encapsulating the sample with a fluorescent dye into water-in-oil emulsion droplets using a microfluidic device, incubating the droplets to allow at least one microorganism to grow, measuring the fluorescence in each droplet, and detecting and/or quantifying at least one population of droplets comprising a microorganism. The method of the invention further allows sorting the droplets depending on their fluorescence intensity and thus separating each microorganism.

Only a small proportion of the microbial biosphere has so far been experimentally explored and characterized in the laboratory by classical cultivation approaches. Well-known major limitations responsible for this are natural competition between organisms differing in growth rates and metabolic capacities in batch cultures, and inhibitory effects due to molecules secreted during microbial growth. Recent progresses in single cell analytical technologies offer new ways of experimentally accessing the still largely unexplored majority of the microbial world.

In current practice, laboratory cultures from environmental inocula containing a wide diversity of organisms with different growth rates and phenotypes will typically result in the enrichment and isolation of the few organisms best suited to the imposed synthetic conditions, and thus overlook highly valuable biological information and material.

Recent progress in single cell analytical technologies offers new ways of addressing this issue, and to better access the still largely unexplored majority of the microbial world, by allowing to isolate each individual of a complex bacterial sample prior to cultivation and analysis. Today, working at single cell resolution is facilitated by using microfluidics to handle very small volumes of liquid in a controlled manner. In particular, droplet-based microfluidics allow extreme miniaturization of reaction vessels down to picoliter (or even femtoliter) volume. With this technology, highly homogeneous water-in-oil droplets produced and manipulated at kHz frequencies can be used as inert individual cell compartments, and allow growth without competition between cells.

Following cell individualization, in-droplet growth can be monitored in several ways. For instance, cell proliferation can be monitored from label-free optical properties. For example, light scattering was used to study antibiotic resistance of bacteria in droplets, although this approach may not be applicable to some types of organisms such as filamentous or slow-growing bacteria. The use of real-time image analysis was also reported, for instance for *Actinobacteria*. However, this approach requires time-consuming image processing, has a relatively low signal to noise ratio, and depends on the position of the microorganism with respect to the focal plane. Similarly, Raman spectroscopy, also proposed for screening of bacterial cultures, requires cell immobilization.

Fluorescence-based methods represent an efficient and sensitive alternative for growth monitoring, including approaches involving genetic modification with a gene coding for a fluorescent reporter such as the Green Fluorescent Protein. For complex communities (e.g., environmental samples), such genetic tools are not applicable, and in this case fluorogenic dyes can be added to droplets to detect growth-associated reactions. For instance, resazurin is reduced to fluorescent resorufin by active metabolism. Unfortunately, resorufin shows poor retention within droplets, and this prevents the fluorescence containment required for subsequent droplet analysis.

There thus remains a genuine need for tools allowing to grow, distinguish and separate mixtures of microorganisms with different growth rates without the biases inherent to batch cultivation.

The present invention is believed to meet such need by providing a method for detecting and/or quantifying at least one microorganism in a sample by encapsulating the sample into a plurality of water-in-oil emulsion droplets and using a non-exchangeable fluorescent dye in the medium.

Indeed, the Inventors have found in a surprising manner that inclusion of a non-exchangeable fluorescent dye in the medium allows following droplet shrinkage and is accurate enough to distinguish microorganisms with different growth rates, to separate and recover them by fluorescence-activated droplet sorting. This method offers a straightforward, high-throughput and non-invasive quantification of microorganisms and their growth.

Thus, the present invention concerns a method for detecting and/or quantifying at least one microorganism in a sample, the method comprising the following steps:

(i) encapsulating the sample into a plurality of water-in-oil emulsion droplets using a microfluidic device, said water-in-oil emulsion droplets comprising a growth medium, a fluorescent dye and a fraction of the sample ;

(ii) incubating the plurality of droplets at a temperature and for a period of time sufficient to allow at least one microorganism to grow for at least two doubling times;

(iii) measuring the fluorescence in each individual droplet, wherein droplets comprising no microorganism have a basal fluorescence intensity and droplets comprising a microorganism have a fluorescence intensity at least equal to 1.001 times the basal fluorescence intensity;

(iv) detecting and/or quantifying at least one population of droplets comprising a microorganism, thereby determining the presence and/or the amount of at least one microorganism in the sample.

As used herein, the expression "microfluidic device" refers to any device or system that allows handling and/or analyzing very small volumes of fluids, e.g. from microliter ($\mu$L) to femtoliter (fL).

As used herein, the expression "fluorescent dye" designates any, natural or artificial, fluorescent compound that can re-emit light upon light excitation.

Preferably, the fluorescent dye is non-exchangeable between the droplets, i.e. it stays confined within droplets and thus becomes more concentrated as the volume of occupied droplets decreases. The retention time of a dye in a droplet is directly correlated with its hydrophobicity: the more hydrophilic the dye, the better its retention.

In an embodiment, the fluorescent dye is chemically modified, rendering it more hydrophilic. Such modification can be useful to reduce or avoid dye leaking from the droplets.

In an embodiment, the fluorescent dye is grafted to a hydrophilic molecule e.g. to a polymer, a peptide or a sugar (such as dextran).

It is advantageous that the fluorescent dye has high extinction coefficient and quantum yield. The extinction coefficient is a measure of the quantity of absorbed light at a given wavelength. Therefore, a high extinction coefficient will lead to a greater amount of light being absorbed. Quantum yield is the number of emitted photons relative to the number of absorbed photons. Preferably, the florescent dye has an extinction coefficient and/or a quantum yield ranging from 0.05 to 1, more preferably close to 1.

It is also advantageous that the fluorescent dye has a negative partition coefficient. The partition coefficient (log D) is a measure of the differential solubility of a compound between two solvents (water and octanol) and therefore reflects the hydrophilicity of the compound. The more negative the logD the better the retention time. Advantageously, logD should be negative for free dyes.

For example, the retention time and logD can be determined as detailed in Woronoff et al. (Analytical chemistry 2011, 83:8, 2852-2857).

It is also advantageous that the fluorescent dye is pH-insensitive, i.e. its fluorescence emission capacity is substantially not affected by pH.

In an embodiment, the fluorescent dye does not interact with the microorganism, i.e. the fluorescence emission capacity of the fluorescent dye is substantially not affected by the growth of the microorganism.

In an embodiment, the fluorescent dye is chosen from the group comprising Alexa Fluor 488, Cy5, ACMS, Cy3, Cy5.5, CF-Dyes (for example CF350, CF488 or CF532), and Oregon Green.

In an embodiment, the fluorescent dye is not sulforhodamine (which can interact with bacteria), rhodamine 6G (which can be exchange by micelles), fluorescein (which is pH-sensitive), coumarin (which leaks from droplets and has antibiotic effects), resorufin (which leaks from droplets) or Atto fluorophores (which are pH-sensitive).

As used herein, the expression "growth medium" or "culture medium" refers to any liquid medium that supports the growth of a population of microorganisms. Different media can be used by one skilled in the art depending on the microorganism(s) to cultivate.

Microbiological media (e.g. M9 medium, 2YT medium, ...) are preferred since they are specifically adapted to the growth of microorganisms, such as bacteria or yeasts.

In an embodiment, the invention relates to a method as defined above, wherein said at least one microorganism is selected from the group consisting of bacteria, yeast, fungi and algae, preferably bacteria.

In an embodiment, the invention relates to a method as defined above, wherein the growth medium used in step (i) has a salt concentration less than 10 g/L, preferably less than 5 g/L.

In an embodiment, the growth medium used in step (i) has a salt concentration from 0 to 10 g/L, preferably from 0 to 5 g/L, more preferably from 3 to 5 g/L.

In an embodiment, the growth medium used in step (i) has a salt concentration of 0, 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5 or 10 g/L.

The less salt there is, the better the osmotic contrast between the drops.

The salt can be any appropriate salt known by the person skilled in the art.

In an embodiment, the salt is chosen from the group comprising NaCl, NaSO$_4$, KCl, Na$_2$SO$_4$, MgSO$_4$, NH$_4$SO$_4$, CaCl$_2$, LiCl, Na$_2$HPO$_4$, NaH$_2$PO$_4$, K$_2$HPO$_4$, KH$_2$PO$_4$ and C$_6$H$_5$Na$_3$O$_7$.

In an embodiment, the growth medium used in step (i) contains at least a compound that prevents or limits the transfer of the fluorescent dye between the droplets.

Such a compound can be any compound known by the person skilled in the art.

This compound can be, but is not limited to, a compound chosen from the group comprising Bovine Serum Albumin (BSA), sugar and a surfactant (such as Pluronic F68 or F127).

In an embodiment, this compound can be a nanoparticle.

In an embodiment, this compound can also be grafted.

In an embodiment, the invention relates to a method as defined above, wherein the droplets obtained from step (i) have a volume from 1 pL to 100 nL, preferably from 1 pL to 10 nL, more preferably 1 pL to 1 nL, even more preferably from 1 pL to 100 pL.

In an embodiment, the invention relates to a method as defined above, wherein the droplets obtained from step (i) have a volume of at least 20 pL.

In an embodiment, the droplets obtained from step (i) have a volume of 1 pL, 5 pL, 10 pL, 50 pL, 100 pL, 200 pL, 500 pL, 1 nL, 5 nL, 10 nL, 50 nL or 100 nL.

In an embodiment, the invention relates to a method as defined above, wherein, in step (ii), the period of time sufficient to allow at least one microorganism to grow for at least two doubling times is at least 15 min, preferably at least 1 hour, more preferably at least two hours.

In an embodiment, the period of time sufficient to allow at least one microorganism to grow for at least two doubling times is from 1 hour to two days, more preferably from 2 hours to 20 hours.

In an embodiment, the period of time sufficient to allow at least one microorganism to grow for at least two doubling times is at least 15 min, 30 min, 1 hour, 2 hours, 3 hours, 5 hours, 10 hours, 12 hours, 24 hours, 48 hours or 72 hours.

In an embodiment, the invention relates to a method as defined above, wherein, in step (iii), the droplets comprising no microorganism have a basal fluorescence intensity and droplets comprising a microorganism have a fluorescence intensity at least equal to 1.002, 1.003, 1.004, 1.005, 1.006, 1.007, 1.008, 1.009, 1.01, 1.02, 1.03, 1.04, 1.05, 1.06, 1.07, 1.08, 1.09, 1.1, 1.15, 1.2, 1.25, 1.3, 1.35, 1.4, 1.45, 1.5, 1.55, 1.6, 1.65, 1.7, 1.75, 1.8, 1.85, 1.9, 1.95 or 2 times the basal fluorescence intensity.

In an embodiment, the invention relates to a method as defined above, wherein said sample is selected from the group consisting of an environmental sample, a food sample, a cosmetic sample, a clinical sample and a sample taken from a surface.

In an embodiment, the invention relates to a method as defined above, wherein said at least one microorganism is a mixed population of two or more distinct microorganisms and the method allows the detection and/or the quantification of each distinct microorganism.

The method of the invention makes it possible to identify and quantify different species, but also different strains of the same species with different phenotypes (e.g. resistance to an antibiotic or antifungal, ability to metabolize a substrate, etc.).

In an embodiment, the invention relates to a method as defined above, wherein the droplets contain an antimicrobial agent and the method allows the detection and/or the quantification of microorganisms resistant or sensitive to the antimicrobial agent.

As used herein, the expression "antimicrobial agent" refers to any natural or synthetic substance that kills or inhibits the growth of microorganisms. The antimicrobial agent can be any antimicrobial agent known by the skilled person in the art.

In an embodiment, the antimicrobial agent is selected from antibiotic compounds (e.g., penicillins, cephalosporins, fluoroquinolones, aminoglycosides, monobactams, carbapenems, macrolides), antifungal compounds (e.g. azoles, polyenes, 5-fluorocytosine), alcohols (e.g. ethyl alcohol) and aromatic oils.

In an embodiment, the invention relates to a method as defined above, wherein the droplets contain a substrate and the method allows the detection and/or the quantification of microorganisms capable to consume the substrate.

The method of the invention is accurate enough to distinguish each droplet and thus allows isolating each microorganism or population via a fluorescence-activated droplet sorting device. Indeed, the fluorescence of each droplet can be analyzed and used for sorting droplets displaying a fluorescence profile of interest.

In an embodiment, the invention thus relates to a method as defined above, wherein said method further comprises a step (v) of sorting the droplets depending on their fluorescence intensity.

The following Examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

**FIGURE LEGENDS**

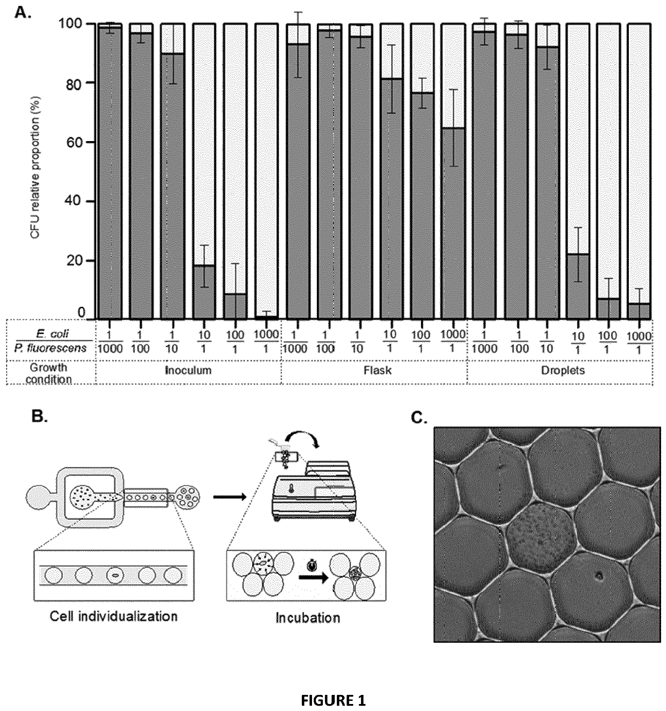

**Figure 1. Bias in growth analysis of model bacterial cultures. A.** Mixtures of *E. coli* and *P. fluorescens* in ratios spanning 7 orders of magnitude were prepared and either inoculated into liquid M9 minimal medium in conventional flasks or dispersed into 20 pL water-in-oil droplets ($\lambda$= 0.2) in the same medium prior to cultivation at 30 °C for 20 hours. The composition of the starting inoculum and that of each culture at the final timepoint was analyzed by plating onto solid M9 minimal medium. Colonies were counted after 48 h incubation and assigned to *E. coli* (small non-fluorescent colonies) or *P. fluorescens* (large fluorescent colonies). Reported values represent the mean of 3 independent experiments, with error bars corresponding to $\pm$ 1 standard deviation. **B.** Microfluidic pipeline for cell individualization and incubation. **C.** Micrograph of picoliter droplets following bacterial growth (scale: 5 $\mu$m).

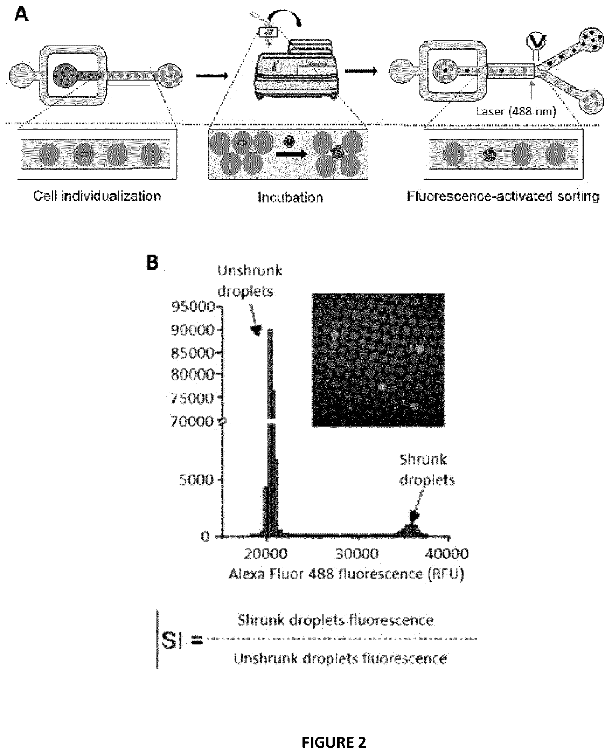

**Figure 2. Shrinkage-based droplet analysis of bacterial growth. A.** Fluorescence-enabled, droplet-based microfluidic screening pipeline. Bacteria are individualized into 20 pL water-in-oil droplets in the presence of the Alexa Fluor 488 fluorophore at 5 $\mu$g/mL final concentration (left), collected, and incubated from 2 to 20 hours at the chosen temperature (middle). Droplets are then re-injected into a fluorescence-activated droplet sorting device (right) in which the fluorescence of each droplet is analyzed and used for sorting droplets displaying the fluorescence profile of interest. **B.** Typical fluorescence profile obtained after incubation. Since not all droplets are occupied by bacteria, at least two distinct populations of droplets can be detected after incubation. Empty droplets conserve their original volume (unshrunk droplets) and show moderate fluorescence (~ 20 000 RFU). Droplets in which bacterial growth took place experience shrinkage (shrunk droplets), leading to an increase in fluorophore concentration and thus in fluorescence intensity (~ 35 000 RFU). Computing the fluorescence ratio of both populations yields the Shrinkage Index (SI, here SI ~ 1.75). The result can be confirmed by epifluorescence microscopy (insert).

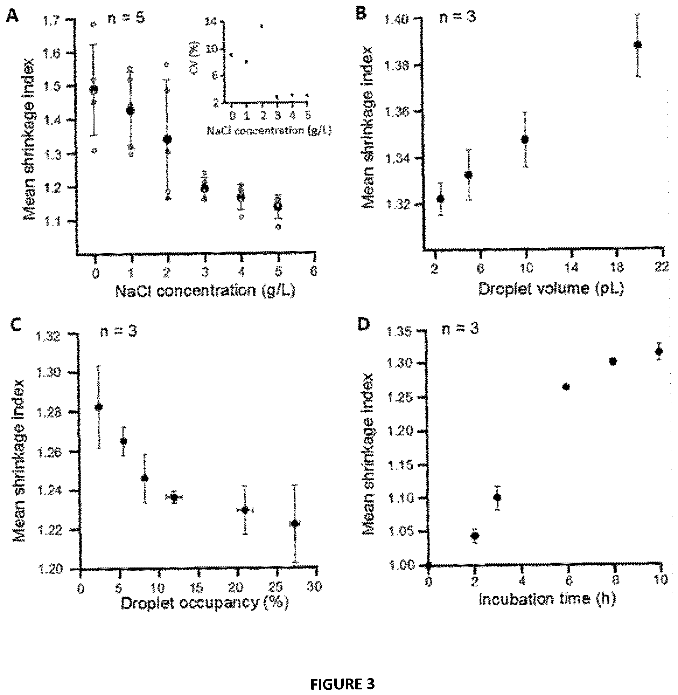

**Figure 3. Main parameters affecting droplet shrinkage.** The Shrinkage Index (SI) was determined as a function of four parameters: NaCl concentration (**A**), with evolution of the calculate Coefficient of Variation (CV) shown as an insert, droplet volume (**B**), droplet occupancy (**C**), and incubation time (**D**). Reported values represent the mean of 5 (grey circles in part A) or 3 (parts B-D) independent replicates, with errors bars corresponding to $\pm$ 1 standard deviation.

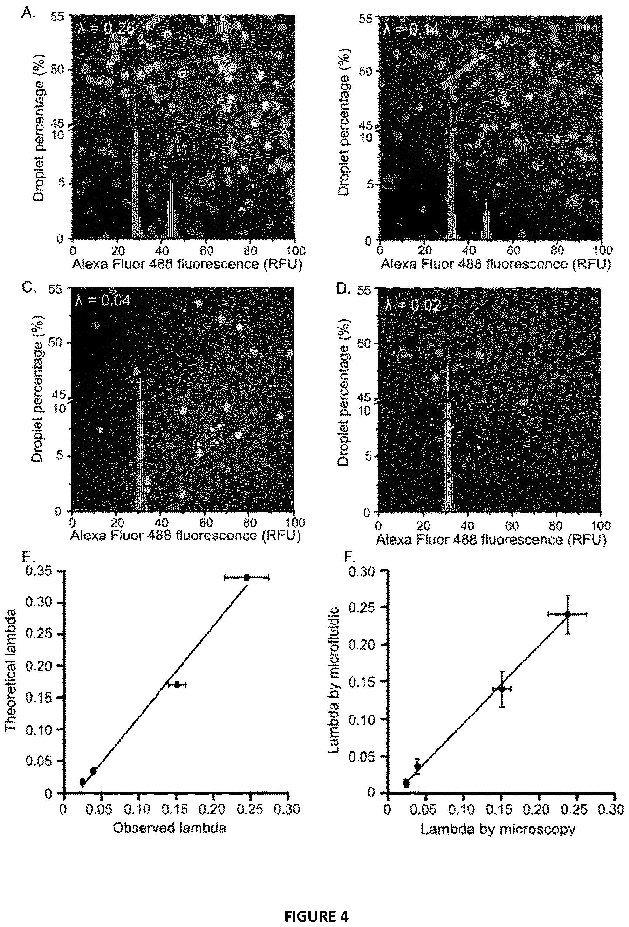

**Figure 4. Droplet-based digital quantification of bacterial suspensions.** Dilutions of *E. coli* were prepared in rich 2YT medium containing 3 g/L NaCl, supplemented with 5 $\mu$g/ml Alexa Fluor 488, and dispersed into 20 pL water-in-oil droplets in the same medium. **A-D:** After 15 hours incubation at 37 °C, the fluorescence of each droplet was either analyzed with a high-throughput optical set-up (bar charts) or evaluated by fluorescence microscopy (micrographs). Average numbers of bacteria per droplet ($\lambda$ values) computed from experimental droplet occupancy using equation 3 are indicated. **E:** correlation between theoretical and observed $\lambda$ values. **F:** correlation between digital high-throughput and microscopic analyses. Values reported in E-F represent the mean of three independent replicates, with error bars corresponding to $\pm$ 1 standard deviation.

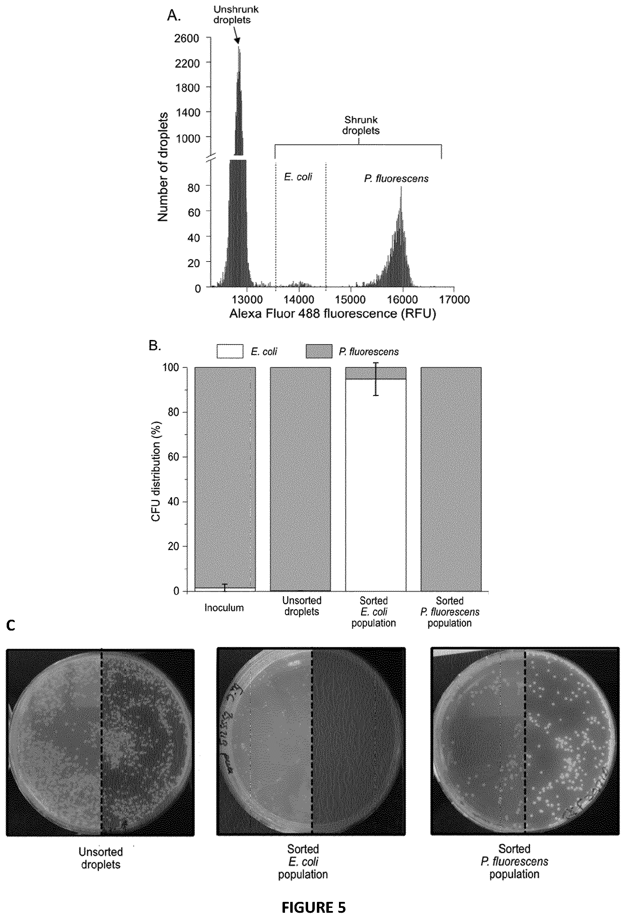

**Figure 5. Microfluidic enabled growth-associated subpopulation enrichment. A.** Fluorescence profile of a model mixed bacterial culture of slow growing *E. coli* and fast-growing *P.fluorescens* in a 100:1 ratio, prepared in minimal M9 medium and supplemented with 5 $\mu$g/ml Alexa Fluor 488 prior to dispersing the mixture in 20 pL droplets, after 20 hours incubation at 30 °C. Two additional populations of droplets were observed, the most abundant, displaying higher fluorescence (~ 16,000 RFU) clearly distinguishable from a minor, less fluorescent (~ 14,000 RFU) population. **B.** Droplets sorted in A were plated on M9 minimal solid medium, and the nature of each colony was assigned to *E. coli* or *P. fluorescens* according to morphological and phenotypic features (see part C). Shown values represent the mean of two independent replicates, with error bars corresponding to $\pm$ 1 standard deviation. **C.** Slower-growing *E. coli* forms smaller colonies than *P. fluorescens* (left-hand side of Petri dishes photographed in normal light), and larger colonies of *P. fluorescens* appear fluorescent under UV light (right-and side of Petri dishes).

## EXAMPLES

## Materials and methods

1. Materials

Microfluidic workstation, microfluidic chips (droplet generator and droplet sorter) and collection tubes were designed and prepared as described elsewhere (Bouhedda et al., Methods in molecular biology 2021, 2300, 203-237). 2YT medium consisted of (per L) 10 g yeast extract, 16 g tryptone, and 0 to 5 g NaCl. M9 mineral medium consisted of (per L) 5.6 g $Na_2HPO_4$, 3 g $KH_2PO_4$ and 1 g $NH_4SO_4$. After autoclaving, medium was supplemented with filter-sterilized solutions, affording final concentrations of 0.05% glucose, 20 $\mu$g/L vitamin B1, 1 mM $MgSO_4$ and 0.1 mg/ml L-leucine.

5 g/L casamino acids were also added for inoculum preparation in liquid culture in M9 medium. Solid media contained 15 g/L agar. *Escherichia coli* (Ec) strain DH5 $\alpha$ (NEB 5-alpha electrocompetent *E. coli* #C2989K) was transformed with plasmid pUC18 carrying the ampicillin resistance gene according to the manufacturer's instructions. *P. fluorescens* strain (Pf) was a laboratory stock.

## 2. Bacterial growth measurements

Growth rate ($\mu$, in $h^{-1}$) of strains Ec and Pf were determined from the slope of *In*$OD_{600}$ values in exponential phase through time. For differential growth measurements, starter cultures of each strain were prepared as overnight cultures in M9 medium. Strains Ec and Pf were diluted to the same $OD_{600}$, mixed in different ratios (1:10, 1:100, 1:1000), and used to inoculate 25 mL in flasks of liquid M9 medium or emulsified as described below. Inocula and grown populations (in flasks or in emulsions) were plated onto solid M9 mineral medium supplemented with 15 g/L Bacto agar and 5 g/L casamino acids and incubated for 48 hours at 30 °C. Colonies were counted and Ec and Pf strains were differentiated basing on colony size (small Ec and large Pf colonies) on solid M9 medium, and fluorescence of Pf upon exposure to 312 nm UV light.

## 3. Bacteria cultivation in droplets

Samples (1 mL) from starter cultures obtained by overnight growth in M9 or 2YT liquid medium under agitation (150 rpm) were centrifuged for 5 min at 8000 rpm, resuspended in fresh medium, and their OD measured at 600 nm (Jenway 7200 visible spectrophotometer). Cell suspensions were diluted in fresh medium to the expected droplet occupancy. For experiments in 20 pL droplets, bacterial suspensions were diluted in medium supplemented with 20 $\mu$g/ml Alexa Fluor 488 (Invitrogen) to an $OD_{600}$ of 0.005, affording a A-value of 0.05. For other values of $\lambda$ and droplet volume, dilutions were adapted accordingly.

Obtained diluted suspensions were transferred to 500 $\mu$l Eppendorf tubes containing a 5x2mm stirrer bar (PTFE Stirrer Bar, Cowie) and sealed by a polydimethylsiloxane (PDMS) plug as described for collection tubes in Bouhedda et al. (Methods in molecular biology 2021, 2300, 203-237). Bacteria were kept in suspension by stirring using a magnetic stirring plate placed next to the tube. The Eppendorf tube was connected to a Fluigent injection pump on one side, and to the microfluidic chip on the other side. The diluted cell suspension was then dispersed in 2.5 to 20 pL water droplets by infusing into the device a stream of Novec-7500 fluorinated oil (3M) supplemented with 3 % fluorosurfactant synthesized in-house. Infusion rates of bacterial suspension and oil phase were adjusted to yield droplets of the wished volume. Emulsions were collected in tubes prior to transfer to a dry bath (Eppendorf ThermoStat C) set at 30 °C or 37 °C for the desired incubation time.

## 4. Droplet analysis and sorting

Following incubation, droplet emulsions were reinjected into a custom droplet fluorescence analysis and sorting device as reported previously (Bouhedda *et al.,* 2021). Droplets were spaced by a stream of surfactant-free Novec-7500 oil and their green fluorescence individually analyzed using an optical set-up (Bouhedda *et al.,* 2021). Both green fluorescence intensity and total droplet fluorescence content (integration of whole droplet fluorescence) were measured and used to compute shrinkage index (see equation 2 below). For sorting, droplets of interest were deflected into the sorting channel by applying a 1200 V and 30 kHz AC electric field. Sorted droplets were recovered by addition of Novec-7500 and enrichment was controlled by plating recovered bacteria on M9 medium supplemented with casamino acids and analyzing colony morphology and fluorescence.

Droplet fluorescence was also analyzed using epi-fluorescence microscopy by deposition of droplets on a glass-slide and imaging on TiE (Nikon) platform equipped with a Lumencor LED light source. Bright-field and green fluorescence (excitation at 470 nm/emission at 490-530 nm) pictures were collected using an Orca-Flash4 (Hamamatsu Photonics K.K.) camera.

## Example I: Growth in droplets helps to preserve the original bacterial diversity in samples

A key challenge for characterization of complex microbial populations (e.g., environmental samples) using cultivation approaches relates to competition for resources between organisms with unequal growth rates and exposure to bacterially produced growth-inhibitory compounds.

A simple two-strain model synthetic microbial community consisting of *Escherichia coli* and *Pseudomonas fluorescens* was investigated. The two strains are characterized by slightly different growth properties. *E. coli* grew best at 37°C, while optimal growth of *P. fluorescens* was at 25-30 °C in both rich and minimal media, with growth rates differing by approximately 30 % at 30 °C in M9 minimal medium. These rather modest differences were enough to strongly favor growth of *P. fluorescens* in flask co-cultures of the two strains, significantly altering the final composition of the mixed culture. As shown in Figure 1A, flask incubations of both strains mixed in ratios spanning 7 orders of magnitude (i.e., from 1/10 to 1000/1, *E. coli/P. fluorescens*) in flasks containing M9 minimal medium for 20 hours (corresponding to ˜ 14 and 20 generations for *E. coli* and *P. fluorescens* respectively) strongly biased the composition of the final culture compared to the relative composition expected from the growth rates of the two strains determined in pure cultures of each strain.

It was assumed that individualizing bacteria prior to growth should reduce the bias observed in traditional flask cultures by limiting cell-to-cell competition. Droplet-based microfluidics allows to encapsulate objects such as cells in highly homogeneous picoliter-size water-in-oil droplets at very high frequency (production of hundreds to thousands of droplets per second). Starting from an initial suspension, cells will distribute in droplets according to Poisson statistics. It is thus possible to precisely predict the fraction of droplets occupied by one bacterium or more. For instance, for an average concentration of 0.2 bacterium per droplet (i.e., 1 bacterium every 5 droplets), one can compute that 16 % of the droplets will be occupied by one bacterium and 2 % by more than 1 by solving equation (1):

$$P_{(X=k)} \ = \ \frac{e^{-\lambda}}{k!} \lambda^k \qquad (1)$$

where $\lambda$ is the average number of bacteria per compartment, k is the exact number of bacteria per compartment, and $P_{(x=k)}$ is the probability of having k bacteria per compartment. With this in mind, *E. coli/P. fluorescens* suspensions prepared in different ratios in minimal M9 medium were emulsified at a $\lambda$ value of 0.05 (4.9 % of occupied droplets) prior to dispersing them into 20 pL water-in-oil droplets. Emulsions were collected and incubated separately for 20 hours at 30 °C (Figure 1B). Growth was observed with individualized bacteria developing monoclonal suspensions confined within the droplets (Figure 1C). Droplets were then retrieved, and the bacterial content of cultured emulsions analyzed on agar plates. Most strikingly and unlike what was observed for flask-based cultivation, cultures grown in this way largely preserved the composition of the starting inoculum (Figure 1A).

This confirmed that an approach exploiting droplet microfluidics represents an efficient way of amplifying the biomass in a sample while limiting biases (and associated information loss) due to competition between microorganisms in conventional batch cultures.

### Example II: Detection of bacterial growth from droplet shrinkage

It was noticed that bacterial growth in droplets was associated with significant droplet size reduction (Figure 1C). This had already been observed previously for both bacteria and yeast, and attributed to droplet-to-droplet water exchange as a consequence of cell growth, nutrient consumption by growing cells generating an osmotic gradient driving water transfer from occupied droplets to empty ones. It was hypothesized that this phenomenon may offer a new approach to determine cell counts in a sample in a high throughput manner, simply by adding a fluorescent dye to the medium (Figure 2).

Choice of the proper reporter dye was crucial. The dye should stay confined within droplets and become more concentrated as the volume of occupied droplets decreases. Previous work showed that retention time of a dye in a droplet is directly correlated with its hydrophobicity: the more hydrophilic the dye, the better its retention. The dye Alexa Fluor 488 was chosen because i) its 2 sulfone groups are known to drastically favor droplet retention; ii) its high extinction coefficient and a quantum yield close to 1; and iii), its pH-insensitive fluorescence, preventing any interference from growth-associated medium acidification.

A suspension of *E. coli* was prepared at $\lambda$ = 0.26 in 2YT rich medium supplemented with Alexa 488 prior to being dispersed into droplets (Figure 2A). The resulting emulsion was collected, incubated for 20 h at 37 °C and re-injected into an analysis and sorting device in which the Alexa Fluor 488 green fluorescence of each droplet was analyzed. As expected, droplet size reduction resulting from bacterial growth led to an increase in fluorescence intensity in the corresponding droplets (Figure 2B). Two distinct populations of droplets were observed, with shrunk droplets (average fluorescence of ˜ 35,000 RFU) readily distinguishable from unshrunk droplets (˜ 20,000 RFU). Of note, the two droplet populations displayed distinct maximum fluorescence, but shared the same total fluorescence, confirming that both types of droplets contained the same total amount of dye, and that only its concentration changed upon droplet shrinkage.

This approach thus affords non-invasive, high throughput analysis (300 occupied droplets analyzed per second) of cells in a sample, and the use of a fluorescent reporter compatible with common filter sets also allows to define the fraction of droplets displaying bacterial growth using fluorescence microscopy (Figure 1B, insert).

**Example III: Optimization of droplet shrinkage**

The potential of droplet shrinkage for detection and quantification of bacterial growth was explored in more detail. In essence, water exchange between droplets is mainly driven by differences in solute concentrations. Specifically, the peptone and tryptone in 2YT medium are consumed during bacterial growth, and their concentration should therefore decrease in droplets where bacterial growth takes place. Conversely, the concentration of NaCl in droplets should remain essentially the same. It was thus hypothesized, however, that salt may interfere with droplet shrinkage by masking growth-dependent changes in the concentration of other solutes.

To test this hypothesis, bacterial suspensions were prepared in 2YT medium supplemented with Alexa Fluor 488 and containing different concentrations of NaCl up to 5 g/L NaCl prior to being emulsified at $\lambda = 0.2$ in 2.5 pL droplets, and incubated for 20 hours at 37 °C. Droplets were then reinjected into an analysis device and their individual fluorescence intensity measured to compute the Shrinkage Index of each emulsion (Figure 2), using equation (2):

$$\text{Shrinkage Index} = \frac{\text{Fluorescence}^{\text{Shrunk droplets}}}{\text{Fluorescence}^{\text{Unshrunk droplets}}} \quad (2)$$

As anticipated, the lower the NaCl concentration, the larger was the observed shrinkage. In contrast, bacterial growth rate remained essentially unaffected at different concentrations of NaCl. However, lowest NaCl concentrations (i.e., from 0 to 2 g/L), while most effective, were also associated with an important variability in the observed shrinkage (Figure 3A, insert), the origin of which remains to be better understood. From 3 g/L NaCl upwards, shrinkage was much more reproducible, and this concentration was then used.

It was expected that beyond droplet occupancy, the extent of surface contact between occupied and bacteria-free droplets also plays a role in the magnitude of droplet shrinkage. To test this hypothesis, shrinkage for droplet volumes ranging from 2.5 pL to 20 pL (i.e., droplet diameters ranging from ~17 to~34 μm respectively) was evaluated. Indeed, a direct correlation was found between droplet size and shrinkage index (Figure 3B). On this basis, it also seemed likely that the more an occupied droplet is surrounded by empty ones, the more exchange will be favored, and the more efficient the shrinkage will be. Indeed, varying droplet occupancy from 2.5 % to 27.5 % led to the expected decrease in shrinkage index (Figure 3C).

As a last optimization, the time required to observe a detectable effect in droplet size, as a crucial parameter for rapid identification of the presence of a live target bacterium (e.g., a pathogenic strain) in a sample, was investigated. Accordingly, and using optimized parameters of salinity and droplet volume and occupancy (3 g/L NaCl, 20 pL droplets at $\lambda = 0.05$), a bacterial suspension in 2YT medium supplemented with Alexa Fluor 488 was emulsified, collected, and its shrinkage index determined at incubation times ranging from 2 to 10 hours (Figure 3D). Under these conditions, droplets containing dividing bacteria were readily identified after an incubation time as short as 2 hours. Shrinkage continued to increase with time, entering a plateau after 8 hours.

**Example IV: Digital titration, analysis and sorting of bacterial suspensions**

Crucially, the microfluidic-assisted approach described here can be used to rapidly and precisely quantify cells in a sample in a universal and non-invasive manner, by exploiting the same principles as digital droplet PCR. As for any digital method, its sensitivity and precision are directly correlated with the number of compartments that can be analyzed, typically several millions of droplets in the present work.

Here and in order to evaluate the potential of this approach to precisely quantify cell counts in bacterial suspensions, dilutions of *E. coli* corresponding to theoretical $\lambda$ values ranging from 0.017 to 0.34 (i.e., theoretical droplet occupancies ranging from 1.7 to 29 %) were prepared. Suspensions were emulsified in 20 pL droplets consisting of 2YT medium supplemented with Alexa 488 and containing 3 g/L NaCl, and then incubated for 15 hours at 37 °C. Droplet fluorescence was then analyzed either by reinjecting droplets into a fluorescence analysis device or by fluorescence microscopy (Figure 4). In both cases, droplet-containing bacteria were readily distinguishable from empty droplets (Figure 4, panels A-D). Experimental $\lambda$ values were computed from observed droplet occupancies using equation (3):

$$\lambda = -\ln(1 - \text{occupancy}) \quad (3)$$

These values were found to be in excellent correlation (Pearson coefficient = 0.99) with those expected from the theory (Figure 4E). Moreover, online fluorescence measurements and microscopy yielded closely similar values (Pearson coefficient = 0.99, Figure 4F), suggesting that a simple common epifluorescence microscope is sufficient to quantify cell counts from a sample using this method. However, online detection will be better suited for more accurate and automated measurements, or for more complex downstream operations involving droplet sorting.

In this context, it was sought to provide proof-of-principle of the potential of our approach for quantification and sorting efficiency of different populations within a bacterial sample.

In order to do this, a 1:100 *E. coli:P. fluorescens* mixed culture was diluted in minimum M9 medium supplemented with Alexa Fluor 488 and incubated at 30°C for 20 hours. Three populations of droplets were readily distinguished and sorted (Figure 5A). The least fluorescent population (~ 12,500 RFU) was associated to empty unshrunk droplets, whereas the two others were predicted to represent droplet suspensions specific of each strain. Given that *E. coli* showed slower growth under the used conditions, it was tentatively assigned to the droplet suspension with an intermediate fluorescence increase (~ 14,000 RFU). The most fluorescent population, i.e., that involving the largest droplet shrinkage (~ 16,000 RFU), was predicted to correspond to droplets containing the best growing bacteria, here *P. fluorescens* (note that fluorescent pyoverdine produced by *P. fluorescens* was not detectable on our optical set-up, confirming that the detected fluorescence increases solely originated from Alexa Fluor 488 as a consequence of droplet shrinkage).

The identity of the different droplet populations was experimentally confirmed by sorting each droplet population using a fluorescence-activated droplet sorting device, prior to recovering bacteria and plating them on solid medium (Figure 5B). As before, each colony was readily assigned to *E. coli* or *P. fluorescens* by comparison of colony morphology and fluorescence under UV light (Figure 5C). *P. fluorescens* represented 98.8% of the total occupied droplets, a value close to the 1/100 ratio of *E. coli* to *P. fluorescens* used as inoculum. Besides, bacteria recovered from sorted droplets were highly enriched in their respective strain, a feature especially interesting in the case of the slow growing *E. coli* strain, which in batch mixed cultures together with *Pseudomonas fluorescens* would have been lost for this reason (see Fig. 1A middle panel).

Incidentally, this last data clearly indicates that this method is not only useful in allowing to detect bacterial growth. It is also quantitative to some extent: allowing to distinguish bacterial populations with different growth rates, it also affords the possibility not only to separate them based on their differences in growth rate, but also of recovering and enriching otherwise underrepresented strains, including slow growers.

## Conclusions

These results provide proof-of-principle for a novel bacterial cultivation and characterization approach, in which bacteria are first individualized in microfluidics-generated picoliter-size water-in-oil droplets. This method allows biomass production while minimizing cultivation bias in samples containing different organisms. The oil surrounding aqueous droplet reactors isolates each cell with its own pool of nutrients, thereby ensuring access of slow growers to nutrients, and limiting competition for resources. Cell compartmentalization also prevents diffusion of molecules secreted by bacteria that may inhibit growth.

Moreover, the observation that droplets occupied by cells decreased in size and volume upon bacterial growth was applied for the rapid detection (as short as two hours) and monitoring of potentially any bacterial cell type in a high-throughput and non-invasive way, by inclusion in the medium of an inert and non-diffusible fluorescent dye as demonstrated in this work for both rich and minimal synthetic media. In effect, monitoring of droplet shrinkage provides access to several digital microbiology approaches, such as quantification of cells in a sample by a simple yes/no (0/1) answer.

Of note, this method only requires a microfluidic droplet generator to ensure proper emulsion monodispersity and limit droplet volume variations. Several benchtop devices and microfluidic chips are now commercially available for this purpose and will facilitate implementation of the proposed method. Further, droplet fluorescence analysis can already be performed with a simple and conventional epifluorescence microscope without the need for advanced analytical equipment, further increasing the accessibility of the proposed approach.

## Claims

1. A method for detecting and/or quantifying at least one microorganism in a sample, the method comprising the following steps:

   (i) encapsulating the sample into a plurality of water-in-oil emulsion droplets using a microfluidic device, said water-in-oil emulsion droplets comprising a growth medium, a fluorescent dye and a fraction of the sample ;
   (ii) incubating the plurality of droplets at a temperature and for a period of time sufficient to allow at least one microorganism to grow for at least two doubling times;
   (iii) measuring the fluorescence in each individual droplet, wherein droplets comprising no microorganism have a basal fluorescence intensity and droplets comprising a microorganism have a fluorescence intensity at least equal to 1.001 times the basal fluorescence intensity;
   (iv) detecting and/or quantifying at least one population of droplets comprising a microorganism, thereby determining the presence and/or the amount of at least one microorganism in the sample.

2. The method according to claim 1, wherein the fluorescent dye is non-exchangeable between the droplets.

3. The method according to claim 1 or 2, wherein the fluorescent dye does not interact with the microorganism and the fluorescence emission capacity of the dye is not affected by the growth of the microorganism.

4. The method according to any one of claims 1 to 3, wherein said at least one microorganism is selected from the group consisting of bacteria, yeast, fungi and algae, preferably bacteria.

5. The method according to any one of claims 1 to 4, wherein the growth medium used in step (i) has a salt concentration from 0 to 10 g/L, preferably less than 5 g/L.

6. The method according to any one of claims 1 to 5, wherein the droplets obtained from step (i) have a volume from 1 pL to 100 nL, preferably of at least 20 pL.

7. The method according to any one of claims 1 to 6, wherein said sample is selected from the group consisting of an environmental sample, a food sample, a cosmetic sample, a clinical sample and a sample taken from a surface.

8. The method according to any one of claims 1 to 7, wherein said at least one microorganism is a mixed population of two or more distinct microorganisms and the method allows the detection and/or the quantification of each distinct microorganism.

9. The method according to any one of claims 1 to 8, wherein the droplets contain an antimicrobial agent and the method allows the detection and/or the quantification of microorganisms resistant or sensitive to the antimicrobial agent.

10. The method according to any one of claims 1 to 9, wherein the droplets contain a substrate and the method allows the detection and/or the quantification of microorganisms capable to consume the substrate.

11. The method according to any one of claims 1 to 10, wherein the growth medium contains at least a compound that prevents or limits the transfer of the fluorescent dye between the droplets.

12. The method according to any one of claims 1 to 11, wherein said method further comprises a step (v) of sorting the droplets depending on their fluorescence intensity.

FIGURE 1

**A**

Cell individualization      Incubation      Fluorescence-activated sorting

**B**

$$SI = \frac{\text{Shrunk droplets fluorescence}}{\text{Unshrunk droplets fluorescence}}$$

**FIGURE 2**

**FIGURE 3**

**FIGURE 4**

FIGURE 5

# EUROPEAN SEARCH REPORT

**Application Number**

EP 22 30 5119

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | OTA YURI ET AL: "Fluorescent nucleic acid probe in droplets for bacterial sorting (FNAP-sort) as a high-throughput screening method for environmental bacteria with various growth rates", PLOS ONE, vol. 14, no. 4, 17 April 2019 (2019-04-17), page e0214533, XP055941960, DOI: 10.1371/journal.pone.0214533 * abstract * * page 2 * * Materials and methods; page 2 – page 5 * * page 13 – page 15 * | 1-12 | INV. C12M1/34 C12M3/06 C12N1/20 C12Q1/04 C12Q1/6888 G01N33/569 |
| X | OTT SCHELER ET AL: "Dodecylresorufin (C12R) Outperforms Resorufin in Microdroplet Bacterial Assays", APPLIED MATERIALS & INTERFACES, vol. 8, no. 18, 29 April 2016 (2016-04-29), pages 11318–11325, XP055687120, US ISSN: 1944-8244, DOI: 10.1021/acsami.6b02360 * abstract * * MATERIALS AND METHODS; page 11323, right-hand column – page 11324, left-hand column * | 1,2,4-12 | |

-/--

TECHNICAL FIELDS SEARCHED (IPC)

C12M
C12N
C12Q
G01N

The present search report has been drawn up for all claims

| Place of search | Date of completion of the search | Examiner |
|---|---|---|
| The Hague | 19 July 2022 | Fabrowski, Piotr |

EPO FORM 1503 03.82 (P04C01)

page 1 of 2

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HAAKAN N JOENSSON ET AL: "Droplet size based seperation by deterministic lateral displacement – separating droplets by cell-induced shrinking", LAB ON A CHIP, ROYAL SOCIETY OF CHEMISTRY, UK, no. 11, 1 January 2011 (2011-01-01), pages 1305-1310, XP002659831, ISSN: 1473-0197, DOI: 10.1039/C01C00688B [retrieved on 2011-02-14] * abstract * ----- | 1-12 | |
| T | GEERSENS ÉMILIE ET AL: "Growth-Associated Droplet Shrinkage for Bacterial Quantification, Growth Monitoring, and Separation by Ultrahigh-Throughput Microfluidics", ACS OMEGA, vol. 7, no. 14, 30 March 2022 (2022-03-30) , pages 12039-12047, XP055941350, US ISSN: 2470-1343, DOI: 10.1021/acsomega.2c00248 ----- | | TECHNICAL FIELDS SEARCHED (IPC) |

The present search report has been drawn up for all claims

| Place of search | Date of completion of the search | Examiner |
|---|---|---|
| The Hague | 19 July 2022 | Fabrowski, Piotr |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WORONOFF et al.** *Analytical chemistry,* 2011, vol. 83 (8), 2852-2857

- **BOUHEDDA et al.** *Methods in molecular biology,* 2021, vol. 2300, 203-237